# EUROPEAN PATENT APPLICATION

(11) **EP 4 079 751 A1**
(43) Date of publication of application: **26.10.2022**
(21) Application number: 21305528.8
(22) Date of filing: 22.04.2021
(51) Int. Cl.: C07K 14/415, C12N 15/82

(54) **IDENTIFICATION OF A NEW GENE INVOLVED IN SEX DETERMINATION IN CUCURBITACEAE**

(71) Applicant: Institut national de recherche pour l'agriculture, l'alimentation et l'environnement, 75007 Paris (FR); Centre national de la recherche scientifique, 75016 Paris (FR)
(72) Inventor: BENDAHMANE, Abdelhafid, 91405 ORSAY (FR); BOUALEM, Adnane, 91405 ORSAY (FR); DOGIMONT, Catherine, 84143 MONTFAVET (FR); TROADEC, Christelle, 91405 ORSAY (FR)
(74) Representative: Gevers & Orès

(57) **Abstract**

The present invention relates to a new gene involved in sex determination in Cucurbitaceae. The loss of function of the protein encoded by this gene induces a transformation of female flowers to hermaphrodite flowers and fruits with non-altered shape.

The present invention is thus directed to Cucurbitaceae plant modified so that expression and/or function of the protein encoded by said new gene is abolished; said plant having hermaphrodite flowers instead of female flowers and preferably fruits with a non-altered shape.

## Description

The present invention relates to a new gene involved in sex determination in Cucurbitaceae. The loss of function of the protein encoded by this gene induces a transformation of female flowers to hermaphrodite flowers and fruits with non-altered shape.

The production of hybrid plants is of great interest in agronomy and in agriculture. In fact, hybrid plants, owing to the phenomenon of heterosis, also called hybrid vigour, display superiority for many characters, relative to the average of their two parents. This superiority may be reflected for example in better vigour, better yield, greater adaptation to the medium in which the hybrid is cultivated, and great uniformity of the hybrid relative to its parents. This hybrid vigour is even greater when the parents are more distant genetically. The creation of pure and stable lines, the future parents of the hybrid, is an indispensable step for creating homogeneous and reproducible hybrid varieties expressing the greatest heterosis. The creation of pure lines involves the self-fertilization of a plant so as to obtain plants having one and the same germplasm, fixed for all of the required characters of productivity, regularity of yield, or of resistance to diseases.

Many dicotyledonous plants, and in particular the Cucurbitaceae, can be gynoecious, androecious, monoecious, hermaphrodite or andromonoecious. The plants having only female flowers are called gynoecious. The plants having only male flowers are called androecious. Androecy is quite a sought-after agronomic character; indeed exclusively male flowers produce more pollen. Besides, the plants having male flowers and separate female flowers but on the same plant are called monoecious. The plants having bisexual flowers are called hermaphrodite and the plants having bisexual and male flowers on the same plant are called andromonoecious.

The family of the Cucurbitaceae includes more than 800 vegetable species distributed in 120 genera in the tropical and subtropical regions. This plant family includes several species of a major agronomic interest and which are cultivated in temperate regions such as the cucumber (*Cucumis sativus*)*,* the melon (*Cucumis melo*), the watermelon (*Citrullus lanatus*)*,* the zucchini (*Cucurbita pepo*) or the pumpkin (*Cucurbita maxima*).

To facilitate the creation of hybrids, there is therefore a need for a system which would enable to control the development of the floral type of a plant of the Cucurbitaceae family and to obtain a plant of a determined floral type.

The Cucurbitaceae have served as a model for the study of sex dimorphism for decades. In the cucumber (*Cucumis sativus*)*,* a monoecious plant, sex determinism is genetically controlled by three loci, F (Female), A (Androecious) and M (Monoecious). Using a candidate gene approach, it has been shown that an ACC synthase co-segregated with the locus F (Trebitsh et al., (1997) "Identification of a 1-aminocyclopropane-1-carboxylic acid synthase gene linked to the female (F) locus that enhances female sex expression in cucumber" Plant Physiol 113: 987-995) and that the monoecious plants have a single copy of this gene while gynoecious plants have an additional copy, the *CsACS1G* gene. Boualem et al. in "A conserved ethylene biosynthesis enzyme leads to andromonoecy in two cucumis species" (PloS ONE, July 2009, Vol. 4, Issue 7, p.1-10) demonstrated that the gene *CsACS2* of *Cucumis sativus* co-segregates with the Monoecious (M) locus. They also showed that *CsACS2* is mainly expressed in female and hermaphrodite flowers. The loss of activity of that enzyme caused the transition from monoecia to andromonoecia.

In the melon, another model Cucurbitaceae, sex determinism is controlled by two loci, the locus A (andromonoecious) and the locus G (gynoecious). The nature of the locus A, responsible for the transition from the monoecious towards the andromonoecious sex type, has been revealed by Boualem et al. in "A Conserved Mutation in an Ethylene Biosynthesis Enzyme Leads to Andromonoecy in Melons" (Science, 321, 836. 2008). This publication has demonstrated that the A gene encoded for an ACC synthase, *CmACS-7.* In the PCT international application published under No. WO2007/125264, the Inventors demonstrated that the allele (A) of *CmACS-7* controls the andromonoecious character of plants, and that the allele (G) controls the gynoecious character of plants. They showed that the loss of function of *CmACS-7* was the cause for the appearance of andromonoecia. The recessive allele g, in combination with the allele A, causes the development of unisexual female flowers or of hermaphrodite flowers, when combined with the allele *α*. Martos-feuntes et al. ("Pleiotropic effects of CmACS7 on fruit growth and quality parameters in melon (Cucumis melo)" Acta Hortic. 2017, 1151) demonstrated that the mutation in *CmACS-7* gene alters fruit shape by controlling fruit shape parameters.

The Inventors in the PCT international application published under No. WO2010/012948 describes in *Cucumis melo* that the two alleles (G) and (g) differ from one another by different levels of a new protein, the protein CmWIP1. Martin et al. ("A transposon-induced epigenetic changes leads to sex determination in melon" Nature, 2009, 461(7267):1135-8) demonstrated that plants with allele G have a higher level of protein CmWIP1 that plants with allele g. Eleblu et al. ("The gynoecious CmWIP1 transcription factor interacts with CmbZIP48 to inhibit carpel development" Scientific reports 9, 15443, 2019) demonstrated that CmWIP1 inhibits carpel development leading to the development of unisexual male flowers.

For breeding application, cucumber is an important economical vegetable crop. Gynoecy has been shown to be positively correlated with the production, and elongated fruit shape is an important determinant of marketable yield. The association of fruit shape and sex expression is a very interesting phenomenon in cucumber. The majority of cucumber cultivars are monoecious or gynoecious, and their fruits usually show elongated shape. However, the fruits developed from perfect flowers on andromonoecious or hermaphroditic plants are round. Similar observations are also well documented in melon (Loy JB, "Fruit size in melon in monoecious and andromonoecious isolines" Cucurbit Genetics Cooperative Report 2006 N° 28-29, pp 12-13). It is known that this association of bisexual flower and altered fruit shape is due to the pleiotropic effects of the monoecy locus (m in cucumber and *α* in melon) rather than due to its close linkage with a fruit shape gene.

Tan et al. in "A novel allele of monoecious (m) locus is responsible or elongated fruit shape and perfect flowers in cucumber (Cucumis sativus L.)" (Theor. Appl. Genet., 2015, 128: 2483-2493) have identified an andromonoecious cucumber line H38 with bisexual flowers and elongated fruits. This is due to the deletion in *CsACS2* encoding a truncated loss-of-function protein. But they also demonstrated that the elongated fruits are not found in the F2 population (crossing hermaphrodite H34 and andromonoecious H38). Even the fruits from a same plant showed different shapes. The Authors conclude that the action of the m locus on fruit shape might not depend on the enzymatic function of the *CsACS2* gene in cucumber. To create pure lines, it is therefore necessary to use plants whose sexual type permits self-fertilization, for example hermaphrodite plants. In Cucurbitaceae, previous studies have shown that the shape of fruits from hermaphrodite flowers have not the shape required by the market.

Considering these drawbacks, the Inventors have further studied the relationship between sex determination and fruit shape in Cucurbitaceae.

The Inventors have identified a new gene *CmHB40* of *Cucumis melo* (SEQ ID N°1). Surprisingly, plants with a loss of function of CmHB40 develop hermaphrodite flowers instead of female flowers without any negative impact on the shape of fruits. The obtained plants may thus be andromonoecious or hermaphrodite.

Notably, the Inventors have shown that when the gene *CsHB40* of *Cucumis sativus,* orthologous of *CmBH40,* is inactivated, the female flowers become hermaphrodite and the wild type elongated shape of fruits is maintained.

The present invention thus relates to a plant belonging to the Cucurbitaceae family harboring a protein (i) having at least 70%, preferably at least 75%, 80%, 85%, 90%, 95%, 98% or 100% identity with the sequence SEQ ID N°3 and (ii) being preferably expressed in the stamen primordia of female flowers at flower development stages 5 and 6 (corresponding to bisexual stage of flower development), said plant being characterized in that:
- expression and/or function of said protein is abolished by genetic engineering techniques, and
- said plant has hermaphrodite flowers instead of female flowers and preferably fruits with a non-altered shape.

Analysis of the expression of a gene in the stamen primordia of female flower may be performed by laser microdissection of stamina primordia, extracting RNA and analyzing the expression of the target gene using Real-Time Quantitative Reverse Transcription PCR; said localized expression may also be observed by *in-situ* hybridization of mRNA to be assessed with a labelled antisense probe as described in Boualem et al., "A cucurbit androecy gene reveals how unisexual flowers develop and dioecy emerges" (Science, 2015, 350; 6261: 688-691).

Flower developmental stages are described in "Developmental analyses reveal early arrests of the spore-bearing parts of reproductive organs in unisexual flowers of cucumber (*Cucumis sativus L.*)*"* (Su-Lan Bai, et al., Planta, 2004, 220: 230-240).

The term "genetic engineering techniques" means in the sense of the present invention all the techniques for manipulating the genome of a living being so as to modify its genotype and consequently its phenotype.

The term "abolish the function of the protein" means that the protein having at least 70% identity with the sequence SEQ ID N°3 that belongs to the homeodomain leucine zipper transcription factor family, losses its function and is not anymore able to link to its DNA target or to activate transcription of target genes.

Genetic engineering techniques to abolish function of a protein are well known by the person skilled in the art and include mutagenesis, such as, ionizing radiation (X-ray, Gamma rays and neutrons); chemical mutagenesis; genome editing with site-directed nucleases (SDNs), e.g., using clustered regularly interspaced short palindromic repeat (CRISPR)-directed nucleases, transcription activator-like effector nuclease (TALENs), zinc-finger nucleases (ZFNs) or meganucleases; and targeting induced local lesions in genomes (TILLING).

"TILLING" is a method of reverse genetics which is based on the ability of an endonuclease to detect mismatches in a double strand of DNA and to create a DNA cleavage at the unpaired bases (MC Callum et al., 2000, Plant Physiology, Vol. 123: 439-442). TILLING could be also based on direct sequencing of amplicons of mutant lines or full sequencing of mutant lines to identify individual plants harboring induced mutations. This technique makes it possible to detect unique mutation points generated by the exposure of plants to a mutagenic chemical compound (http://ips2.u-psud.fr/en/platforms/epitrans-epigenomic-translational-biology/applications/mutation-screening.html). TILLING technique thus allows the identification of a series of alleles of a given gene and is particularly well suited to the implementation of methods of high throughput screenings for allowing the selection, in the target genes of interest, mutations induced by chemical mutagenesis, like EMS chemical exposition. The chemical mutagenesis may be realized by exposing the plant cells, the protoplasts or the seeds to the EMS (ethyl methanesulfonate, see Koornbeef et al., 1982, Mutat Res, Vol. 93: 109-123), sodium azide or methylnitrourea mutagenic agents.

Mutagenesis can also be carried by a combination of TILLING and gene editing technics as described in Jacob P. et al., 2018 (Translational Research: Exploring and Creating Genetic Diversity Trends in Plant Sciences 23(1):42-52).

According to a specific embodiment, mutagenesis is performed with the TILLING method and the example of primers are listed in table 2 (see example 3).

The loss of protein function could be measured using techniques such as DAPseq (Bartlett, A. et al., "Mapping genome-wide transcription-factor binding sites using DAP-seq", Nat Protoc 12, 1659-1672,2017), ChIP-seq (Park P. "ChIP-seq: advantages and challenges of a maturing technology" Nat Rev Genet 10, 669-680 (2009)), promoter reporter gene such as I-Block (Sarolta S. et al., "I-Block: a simple Escherichia coli-based assay for studying sequence-specific DNA binding of proteins", Nucleic Acids Research, Volume 48, Issue 5, 18 March 2020) or luciferase reporter system as described in Hernandez-Garcia and Finer ("Identification and validation of promoters and cis-acting regulatory elements", Plant Science, Volumes 217-218, 2014, Pages 109-119) or Lin, Yuan et al. ("Rapid validation of transcriptional enhancers using agrobacterium-mediated transient assay." Plant methods vol. 15 21. 6 Mar. 2019). Briefly, in the case of CmBH40 for example, the binding motif "CAATAAT" of the protein of SEQ ID N°3 (CmBH40) or a related motif is inserted in at least one copy upstream of 35S minimal promoter driving Luciferase reporter gene and expressed via *Agrobacterium* binary vector such as pgreen or pCAMBIA vectors. The construct is agroinfiltrated with Agrobacterium binary vector expressing *CmBH40* coding sequence under constitutive promoter such as 35S promoter. Wildtype CmBH40 protein will lead to expression of the reporter gene. Loss of function mutant of CmBH40 will not permit transcription of the reporter gene.

The term "abolish the expression of the protein" means preventing the accumulation of mRNA encoding said protein in the plant.

This low expression could be obtained by further genetic engineering techniques such as gene silencing induced by an interference technics such as RNAi, antisense RNA or aptamers or mutation in the promoter of the protein having at least 70% identity with the sequence SEQ ID N°3.

The accumulation of mRNA encoding the protein can be measured using technics such as qPCR and digital PCR.

The loss of function of the protein having at least 70% identity with the sequence SEQ ID N°3 transforms female flowers into hermaphrodite flowers. This inactivation leads to sexual transition from monoecy to andromonoecy and gynoecy to hermaphroditism.

According to a preferred embodiment, the fruits produced by the plant with the loss of function of the protein having at least 70% identity with the sequence SEQ ID N°3 have a non-altered or normal shape.

The term "normal shape" (also designated as non-altered shape) means the shape of wild type fruit.

One way to assess the shape of a fruit is to calculate the Fruit Shape index (FSi) that is the ratio of the fruit length (FL, i.e. the longer dimension measured from the peduncle) by the fruit diameter (FD, that is the larger dimension in a perpendicular axis compared to the length) at mature stage (see example 6); it is considered that the shape of a fruit is not altered and is thus "normal" in case its FSi does not vary more than 20%, preferably 15%, more preferably 10%, compared to the fruit of a wild type plant.

According to a preferred embodiment, the plant is *Cucumis melo* and the protein has at least 90% identity with the sequence SEQ ID N°3. The protein of SEQ ID N°3 is called CmHB40.

Melon fruits display wide varieties of fruit shapes, varying from round, oblate, ovate, elliptical, or extremely elongated, as in the case of *flexuosus* variety of melon (Antonio J. Monforte et al., "The genetic basis of fruit morphology in horticultural crops: lessons from tomato and melon", Journal of Experimental Botany, Volume 65, Issue 16, August 2014, Pages 4625-4637). The shape of fruits produced by the mutated plant of the invention is similar to the shape of fruits developed from the wild type female flower whatever its initial forms and shapes.

Fruit shape index (FSi), the ratio of the fruit length (FL) by the fruit diameter (FD) at mature stage could be used to measure the roundness of a given fruit. In Charentais-Mono variety, fruits developed by female flowers are elliptical with a FSi score of about 1.8.

According to another preferred embodiment, the plant is *Cucumis sativus* and the protein has at least 90% identity with the sequence SEQ ID N°5. The protein of SEQ ID N°5 is called CsHB40. The shape of fruits produced by the mutated plant is similar to the shape of fruits developed from the initial wild type female flowers, that is to say an elongated shape. In PoinSett variety of cucumber, fruits developed by female flowers are elongated with a FSi of with score of about 4.

According to a preferred embodiment, the plant is *Cucurbita pepo. Cucurbita pepo* genome comprises two copies of *CpHB40,* encoding CpHB40-1 (SEQ ID N°9) and CpHB40-2 (SEQ ID N°10). The inactivation of both of these proteins of SEQ ID N°9 and SEQ ID N°10 is required to lead to hermaphrodite flowers. Accordingly, in *Cucurbita pepo,* it is preferably the expression and/or function of the protein having at least 90% identity with the sequence SEQ ID N°9 and of the protein having at least 90% identity with the sequence SEQ ID N°10 that is abolished.

The shape of fruits produced by the mutated plant is similar to the shape of fruits of developed from the initial wild type female flowers.

According to another preferred embodiment, the plant is *Citrullus lanatus;* and the protein has at least 90% identity with the sequence SEQ ID N°8. The protein of SEQ ID N°8 is called CIHB40. The shape of fruits produced by the mutated plant is similar to the shape of fruits developed from the initial wild type female flowers.

According to another preferred embodiment, the plant is *Lagenaria siceraria;* and the protein has at least 90% identity with the sequence SEQ ID N°11. The protein of SEQ ID N°11 is called LsHB40. The shape of fruits produced by the mutated plant is similar to the shape of fruits of developed from the initial wild type female flowers.

The present invention also relates to seeds of the plants of the invention, in particular to the seeds obtained from plant belonging to the Cucurbitaceae family, such as *Cucumis melo, Cucumis sativus, Cucurbita pepo, Lagenaria siceraria* and *Citrullus lanatus* with expression and/or activity abolished for the protein having at least 70% identity with SEQ ID N°3.

Further the invention relates a method to produce andromonoecious or hermaphrodite Cucurbitaceae plant with normal fruits, wherein the method comprises the following steps:
a) abolition of the expression and/or the function of the protein having at least 70%, preferably at least 75%, 80%, 85%, 90%, 95%, 98% or 100% identity with the sequence SEQ ID N°3 in a protoplast derived from a plant cell or a seed, preferably a seed, by genetic engineering techniques;
b) selection of a protoplast or a seed obtained of step a) with the expression and/or activity of the protein having at least 70%, preferably at least 75%, 80%, 85%, 90%, 95%, 98% or 100% identity with the sequence SEQ ID N°3 that is abolished. Once identified, the plants harboring mutations in SEQ ID N°3 could be also directly phenotyped for sex transition.

Genetic engineering techniques are as defined above.

According to specific embodiments of this method, the plant is *Cucumis melo* and the protein has at least 90% identity with the sequence SEQ ID N°3, the plant is *Cucumis sativus* and said protein has at least 90% identity with the sequence SEQ ID N°5; the plant is *Cucumis pepo* and said protein comprises a protein that has at least 90% identity with the sequence SEQ ID N°9 and a protein that has at least 90% identity with the sequence SEQ ID N°10; the plant is *Citrullus lanatus* and said protein has at least 90% identity with the sequence SEQ ID N°8; or the plant is *Lagenaria siceraria* and said protein has at least 90% identity with the sequence SEQ ID N°11.

According to a particular embodiment, the mutation of the protein having at least 70% identity with the sequence SEQ ID N°3 leads to insertion, deletion and/or substitution of one or more nucleotides in the nucleic sequence encoding for said protein; according to a preferred embodiment of the invention, the mutation leads to a nonsense mutation.

For example, the mutation of the protein of SEQ ID N°3 is a nonsense mutation at amino acid at position 107 or at position 113 (see for example mutant of SEQ ID N°4); the mutations of proteins of SEQ ID N°5, 8, 9, 10 and 11 may also be nonsense mutation of amino acid located at a position corresponding to the position 107 or the position 113 when aligning the protein sequence to SEQ ID N°3 (see for example mutants of *Cucumis sativus* of SEQ ID N°6 and 7). Unless otherwise specified, identity percentages are calculated from a global alignment of amino acid sequences using the "needle" algorithm (Needleman and Wunsch, 1970) using the default settings: "Matrix": EBLOSUM62, "Gap penalty": 10.0 and "Extend penalty": 0.5.

The invention also relates a method for producing a hybrid Cucurbitaceae, wherein the method comprises crossing a plant belonging to the Cucurbitaceae family characterized in that the expression and/or activity of the protein having at least 70%, preferably at least 75%, 80%, 85%, 90%, 95%, 98% or 100% identity with the sequence SEQ ID N°3 is abolished by mutations induced by genetic engineering techniques with a different Cucurbitaceae plant and harvesting the resultant F1 Cucurbitaceae seed.

A further object of the invention is F1 Cucurbitaceae seed produced by the method for producing a hybrid Cucurbitaceae.

As described above, the prior art has shown that mutations in genes involved in sex determination in Cucurbitaceae have a negative impact on the shape of fruits and can consequently not be used in seed industry.

The present invention provides a method to transform female flowers into hermaphrodite flowers in melon, cucumber, watermelon, bottle gourd and zucchini with a resulting plant showing a correct shape of fruits, which refers to non-limiting Figures and Examples illustrating the identification of CmHB40 and its orthologs in accordance with the invention and the demonstration of its impact on sex determination.

### FIGURES

**Figure 1****:** Development of the *M2*/*m2* mapping population
**Figure 2****:** Positional cloning of M2 locus responsible for dominant andromonoecy in melon *(Cucumis melo)*
**Figure 3****:** CmHB40_Wl13STOP mutation in CmHB40 protein leads to andromonoecy in melon
**Figure 4****.** Synteny analysis between the melon MELO3C007809 (coding for CmHB40 protein), the cucumber Csa6G501990 (coding for CsHB40 protein), the watermelon CICG01G019340 (coding for CIHB40 protein), the zucchini Cp4.1LG10G00850 and Cp4.1LG19g09540 (coding for CpHB40-1 and CpHB40-2) and the lagenaria Lsi01G006990 (coding for LsHB40).
**Figure 5****:** Phylogenetic tree of HD-ZIP class I proteins in cucurbits and percentage of identity between HB40 orthologous proteins.
**Figure 6****:** W113STOP and Q107STOP mutations in CsHB40 lead to andromonoecy in cucumber.
**Figure 7****:** Mutations in HB40 in melon and cucumber leads to andromonoecy without altering fruit shape.

### EXAMPLES

### EXAMPLE 1 - Segregation analysis of M2 locus

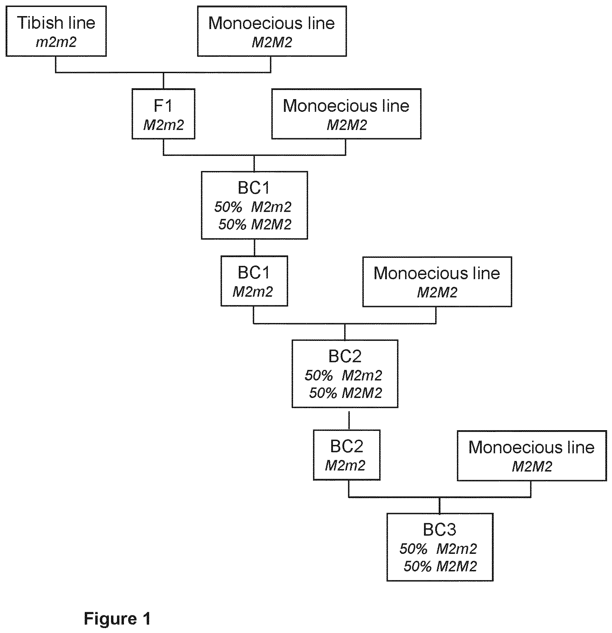

To genetically characterize the locus m2 responsible for dominant andromonoecy in melon *(Cucumis melo),* a segregating population has been generated and the offspring has been phenotyped. To do so, monoecious melon plants (male flowers and female flowers on the same plant) have been crossed with Tibish accession harboring male flowers and hermaphrodite flowers on the same plant. Obtained F1 hybrids were andromonoecious, confirming that Tibish andromonoecy is dominant. The F1 hybrids were then crossed with the monoecious parent to produce the backcross descendant 1 (BC1) plants **(****Figure 1****).** More than 200 BC1 descendant plants were phenotyped for sex determination (monoecy versus andromonoecy); about 50% of the plants were monoecious and 50% were andromonecious, confirming the dominance of the andromonoecy versus monoecy.

### EXAMPLE 2 - Positional cloning of m2 locus controlling dominant andromonoecy in melon

To identify molecular markers linked to m2 locus, bulked-genomic DNA from monoecious and andromonoecious backcross plants were sequenced and the delta-SNP index was determined. This technique is a combination of genome sequencing and Bulk Segregant Analysis (BSA), (Michelmore, R.W. et al. "Identification of markers linked to disease-resistance genes by bulked segregant analysis: A rapid method to detect markers in specific genomic regions by using segregating populations". Proc. Natl. Acad. Sci. USA. 88: 9828-9832, 1991).

Linked polymorphic sequences were converted into CAPS markers (Cleaved amplified polymorphisms). To develop a high resolution genetic map, CAPS markers were mapped relative to m2 locus in a segregating population of more than 3000 plants. In this analysis the inventors mapped m2 locus to a DNA sequence of 11 kb between marker M8 and marker M9 **(Table 1).**

**Table 1: Primer sequence of the genetic markers**

| **Marker Name** | **Primer sequence 5' --> 3'** | **SEQ ID N°** |
|---|---|---|
| M8_F | GAAGGACCCGTAAGAATATAATGTC | 12 |
| M8_R | GGGGCATTTTGCCTAATTTTGGTGA | 13 |
| M9_F | TATTGAGAATGACTGTCACTT | 14 |
| M9_R | CTTCACTATCTTGCCTTTCC | 15 |
| M7809 | AGG CAAACACAAATGG GCATAACAACC | 16 |
| M7809 | GTAATTGTTGTCTTGCATGCA | 17 |
| P7809.1_F | AGTGTGTGAGATGATGGATGATGTG | 18 |
| P7809.1_R | TGCCAAACTTGAGAAAGAGTTG | 19 |
| P7809.2_F | ACAAACCGAACTTTATGAGGTAGGC | 20 |
| P7809.2_R | TGACTCGATGAAATGGGAAGCTTGT | 21 |
| P7809.3_F | GAGAGTGAATCACGTGTCTTGATGT | 22 |
| P7809.3_R | AGTTGGAAATGGAATCCCTTTTTGG | 23 |

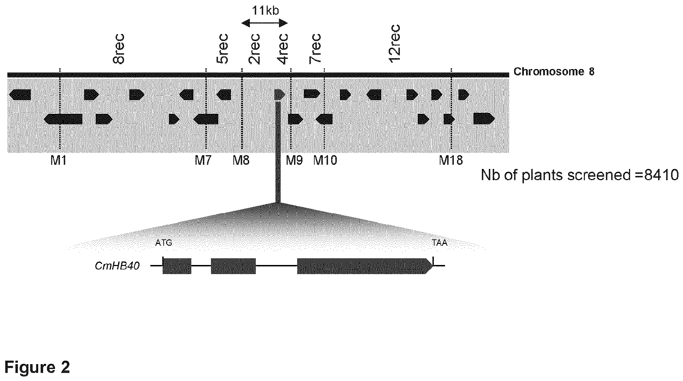

Annotation of DNA sequence located between marker M8 and marker M9 revealed a single gene coding for MELO3C007809 (SEQ ID N°1) **(****Figure 2****).**

### EXAMPLE 3 - Functional validation of the gene MELO3C007809 in melon

To further confirm that MELO3C007809 (SEQ ID N°1) is indeed the gene controlling dominant andromonoecy *versus* monoecy, induced mutations in MELO3C007809 were screened, using the TILLING concept. As control, induced mutations in the two genes flanking MELO3C007809 were also screened. In the TILLING experiment a melon EMS mutagenized population of more than 10 000 M2 families described in Boualem *et al.* 2015, were used. The TILLING screens were also performed, as described in Boualem *et al.* 2015.

To identify induced mutations in MELO3C007809 three amplicons covering all the coding sequence of MELO3C007809 were screened (see **Table 2).**

**Table 2: Sequence of the TILLING primers for melon and cucumber screens**

| **TILLING Primers** | **Primer sequence 5' --> 3'** | **SEQ ID N°** |
|---|---|---|
| Cm7809For139 | AATTCCCGTGTAAGCCATCC | 24 |
| Cm7809Rev142 | CAAAGTTCATCTCCAAAAGC | 25 |
| Cm7809For143 | TACGGGAGAGGGAAATAAGC | 26 |
| Cm7809Rev14S | ACAATTATAGCTCTCTGACC | 27 |
| Cm7809For147 | CACAAATGGGCATAACAACC | 28 |
| Cm7809Rev149 | TATGAGAGTTCTATATCTA | 29 |
| Cs7809For1 | CTCAAACCTATCCACATACC | 30 |
| Cs7809Rev2 | CCTCTCCGATTCCAATTTAT | 31 |
| Cs7809For3 | GGAGATGAATTTTGGGAATG | 32 |
| Cs7809Rev4 | TAACTCTCTGACCGTACGTA | 33 |
| Cs7809For9 | CTGCCATCTGAGGCAAATAT | 34 |
| Cs7809Rev10 | TTAAGCTAGAGATGATCTAA | 35 |

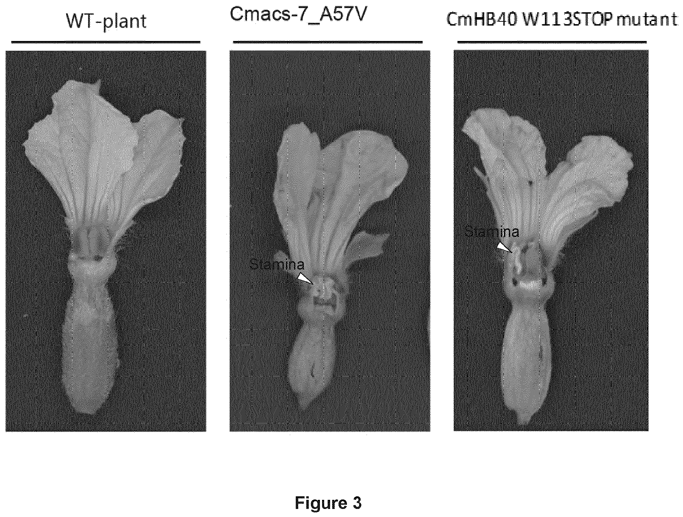

In altogether 8 Missense mutations and one G428A nonsense mutation were identified and the G428A nonsense mutation was found to be located at *428 bp* from ATG start site. G428A stop codon mutation leads to a truncated form of the protein of 113 amino acids instead of a protein of 224 amino acids (SEQ ID N°4). Seeds belonging to the stop mutant family (annotated here by CmHB40_W113STOP*) were sown and resulting plants genotyped using *CAPS* technique, where primers 7809-143For and 7809-145Rev where used to amplify genomic targeted region and Hph1 enzyme was used to detect the polymorphism. Andromonoecious plants homozygote for G428A stop codon mutation were obtained. Conversely plants harboring the wildtype allele are monoecious. These data confirmed that M2 locus encodes for MELO3C007809 gene, the wildtype allele inhibits stamina development and loss of function allele of MELO3C007809 leads to andromonoecy **(****Figure 3****).**

### EXAMPLE 4 - Functional annotation of MELO3C007809 and Identification of functional homologs in cucurbits

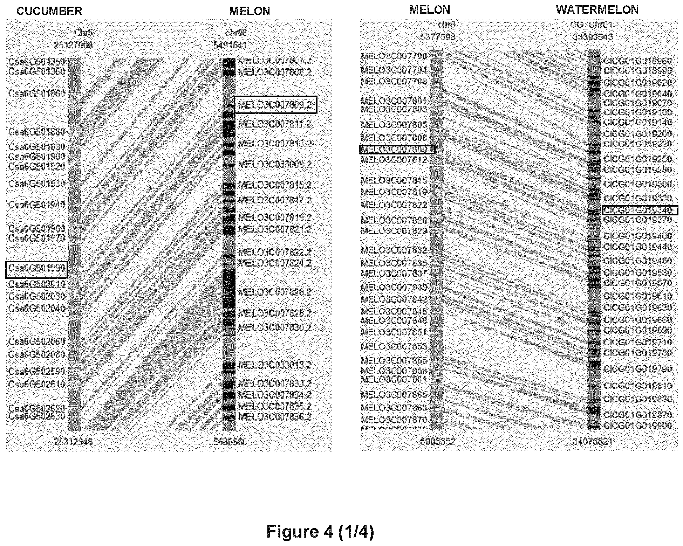

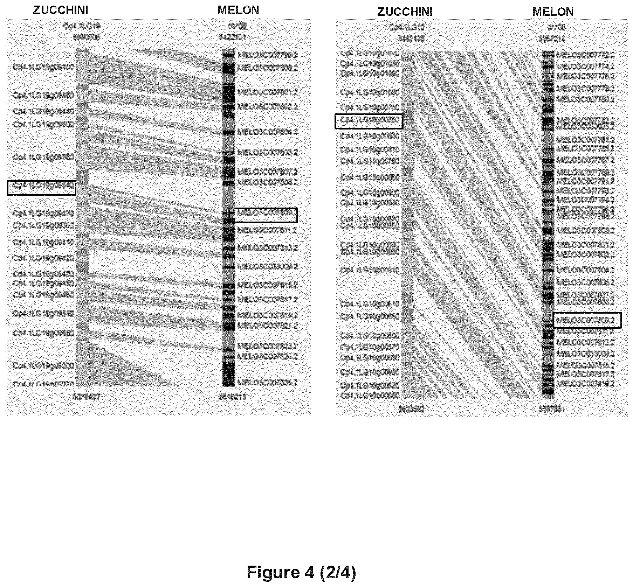

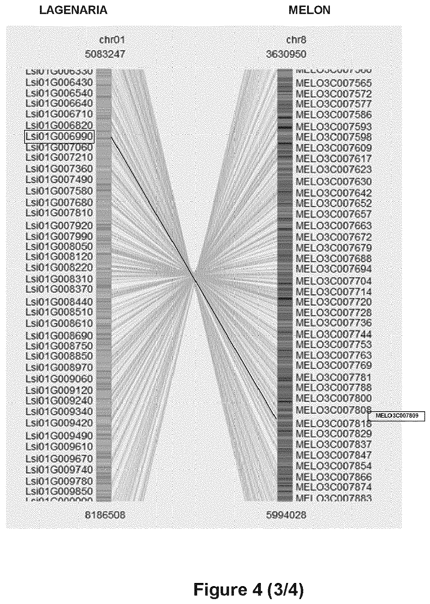

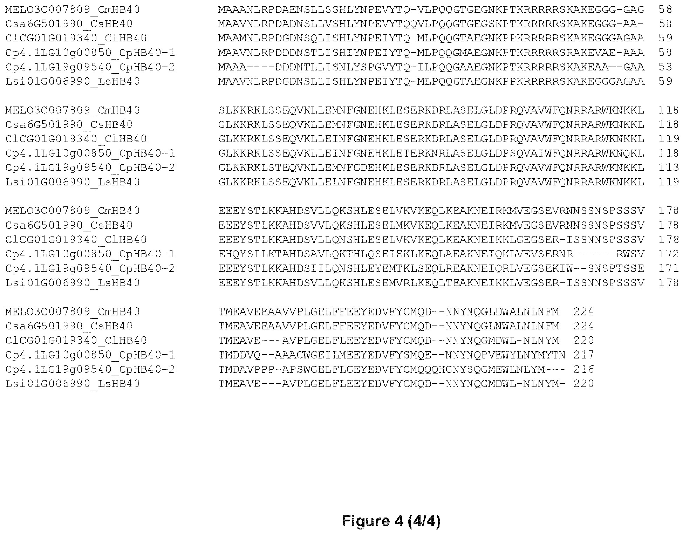

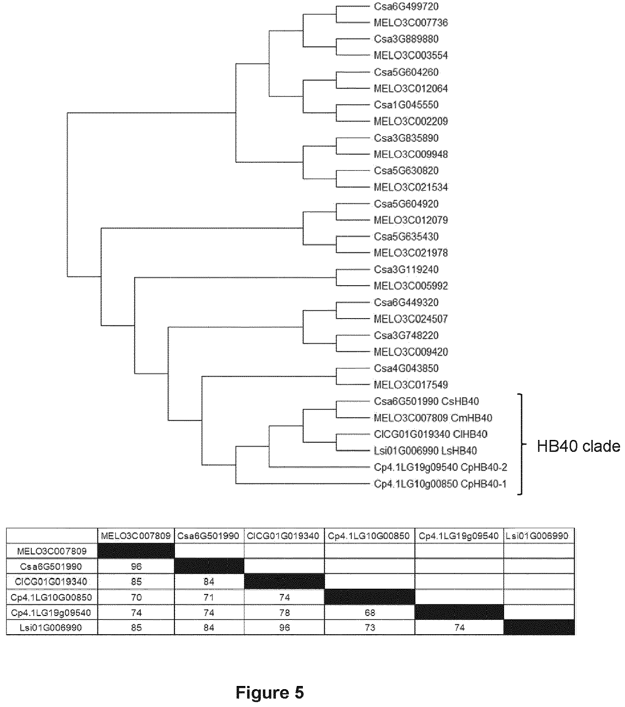

Blasting the sequence of MELO3C007809 gene against *Arabidopsis thaliana* database identified the gene AT4G36740 as the closest homolog. AT4G36740 encodes for a protein belonging to the family of Homeodomain leucine Zipper class I transcription factor. Based on this, MELO3C007809 was re-annotated as Homeodomain leucine Zipper class I gene CmHB40. Blasting the sequence of *CmHB40* gene against the cucurbit genomes identified Csa6G501990, CICG01G019340, Cp4.1LG19g09540, Cp4.1LG10G00850 and Lsi01G006990 as the most homologous sequences in cucumber, watermelon, *Cucurbita pepo* (zucchini) and *Lagenaria siceraria* (bottle gourd), respectively. Using synteny analysis it has been found that MELO3C007809 (coding for CmHB40 protein of SEQ ID N°3), Csa6G501990 (coding for CsHB40 protein of SEQ ID N°5), CICG01G019340 (coding for CIHB40 protein of SEQ ID N°8), Cp4.1LG10G00850 (coding for CpHB40-1 of SEQ ID N°9), Cp4.1LG19g09540 (coding for CpHB40-2 of SEQ ID N°10), and Lsi01G006990 (coding for LsHB40 protein of SEQ ID N°11) are syntenic **(****Figure 4** **and** **Figure 5****).**

### EXAMPLE 5 - Construction of the mutant CsHB40

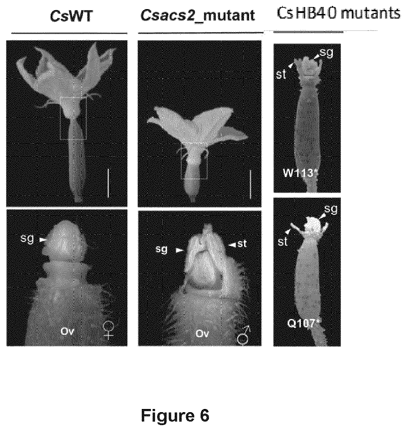

To check whether the function of CmHB40 is conserved throughout cucurbits, the effect of CsHB40 on the control of stamina development in cucumber has been tested. As for melon induced mutations in CsHB40 in cucumber EMS mutagenized population were screened **(Table 2)** and the mutant plants for sex transition were phenotyped. 5 mutations were identified among which two were predicted to lead to nonsense mutations, *Cshb40*-Q107* and *Chb40-*W113**. Cshb40*-Q107* carries a mutation at a position 394 bp from ATG, leading to truncated protein sequence (SEQ ID N°6). *Cshb40-*W113*** carries a mutation at a position 414 bp from ATG, leading to truncated protein sequence (SEQ ID N°7). As in the case of C. *melo,* seeds of these two families were backcrossed to wildtype and F2 seeds sown and plant assessed for sex transition. All the plants homozygote for *Cshb40-*W113*** or *Cshb40*-Q107*** mutations were andromonecious. In contrast sibling plants that do not carrying *Cshb40-*W113* or *Cshb40*-Q107* mutations were monoecious. Based on these findings, it has been concluded that CsHB40 in cucumber, as CmHB40 in melon, inhibits stamina development in female flowers and loss of function mutations release the inhibitions, leading to hermaphrodite flowers **(****Figure 6****).**

### EXAMPLE 6 - Shape of fruits of the mutants CmHB40 and CsHB40

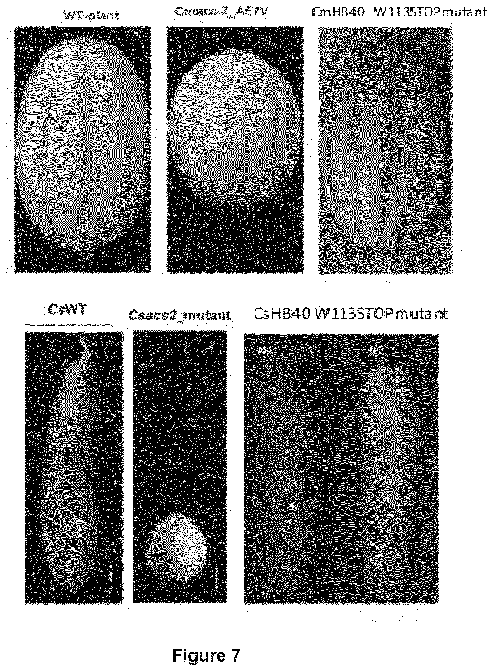

Sex determination genes were shown to be associated to QTL controlling fruit shape. Fruit shape is one of the most important physical properties and quality parameters of all agricultural products. The appearance of fruits and vegetables also has a major influence on the perceived fruit quality, with consumers preferring fruits of uniform shape. It has been found that mutation affecting the enzymatic activity of *CmACS-7* in melon or *CsACS2* in cucumber alter the sex of the flower as well as fruit shape. The inventors tested whether induced mutations in *CmHB40 or CsHB40* leading to andromonoecious phenotype are also associated with alteration of fruit shape. The fruit shape here is referred to as the shape of the fruit developed from wildtype female flower, varying from round, oblate, ovate, elliptical, or extremely elongated, as in the case of *flexuosus* variety of melon. All the mutant plants, harboring mutations in *CmHB40* or in *CsHB40* and displaying sex transition, were found to develop fruits without alteration of fruit shape of the non-mutated wildtype plant. In contrast *CmACS-7 or CsACS2* sex transition mutant developed alteration of fruit shape index **(****Figure 7****).** In Charentais-Mono variety of melon, fruits developed by female flowers are elongated with a FSi of about 1.8. Fruits of CmHB40 mutants have FSi score of about 1.8 and fruits of CmACS7 mutant have FSi score of about 1.4.

In PoinSett variety of cucumber, fruits developed by female flowers are elongated with a FSi of about 4. Fruits of CsHB40 mutants have FSi score of about 4 and fruits of CsACS7 mutant have FSi score of about 1.

## Claims

1. A plant belonging to the Cucurbitaceae family harboring a protein having at least 70%, preferably at least 75%, 80%, 85%, 90%, 95%, 98% or 100% identity with the sequence SEQ ID N°3, said plant being **characterized in that**:
- expression and/or function of said protein is abolished by genetic engineering techniques,
- said plant has hermaphrodite flowers instead of female flowers, and
- the Fruit Shape index (FSi) of said plant does not vary more than 20% compared to the fruit of a wild type plant.

2. The plant according to claim 1 **characterized in that** the plant is *Cucumis melo.*

3. The plant according to claim 2 **characterized in that** said protein has at least 90% identity with the sequence SEQ ID N°3.

4. The plant according to claim 1 **characterized in that** the plant is *Cucumis sativus.*

5. The plant according to claim 4, **characterized in that** said protein has at least 90% identity with the sequence SEQ ID N°5.

6. The plant according to claim 1 **characterized in that** the plant is *Cucurbita pepo.*

7. The plant according to claim 6, **characterized in that** said protein comprises a protein having at least 90% identity with the sequence SEQ ID N°9 and a protein having at least 90% identity with the sequence SEQ ID N°10 and **in that** expression and/or function of both proteins is abolished.

8. The plant according to claim 1 **characterized in that** the plant is *Citrullus lanatus.*

9. The plant according to claim 8 **characterized in that** said protein has at least 90% identity with the sequence SEQ ID N°8.

10. The plant according to claim 1 **characterized in that** the plant is *Lagenaria siceraria.*

11. The plant according to claim 10 **characterized in that** said protein has at least 90% identity with the sequence SEQ ID N°11.

12. The seeds of the plant according to any claims 1 to 11.

13. A method to produce Cucurbitaceae plants according to anyone of claims 1 to 11, wherein the method comprises the following steps:
a) abolition of the expression and/or the activity of the protein having at least 70%, preferably at least 75%, 80%, 85%, 90%, 95%, 98% or 100% identity with the sequence SEQ ID N°3 in a seed by genetic engineering techniques;
b) selection of a seed obtained from step a) wherein the expression and/or activity of the protein having at least 70%, preferably at least 75%, 80%, 85%, 90%, 95%, 98% or 100% identity with the sequence SEQ ID N°3 is abolished.

14. The method according to claim 13, **characterized in that** the genetic engineering technique of step a) is performed by Ethyl Methane sulfonate (EMS) treatment and targeting induced local lesions in genomes (TILLING).

15. The method as claimed in claim 13 or 14 **characterized in that** the abolition of the expression and/or the function of said protein is obtained by a nonsense mutation at amino acid located at position corresponding to position 107 or position 113 of protein of SEQ ID N°3 after alignment.


SEQUENCE LISTING

<110>  Institut national de recherche pour l'agriculture,
       l'alimentation et l'environnement
       Centre National de la Recherche Scientifique

<120>  Identification of a new gene involved in sex determination in
       Cucurbitaceae

<130>  PAT2600233EP00

<160>  35

<170>  PatentIn version 3.5

<210>  1
<211>  2617
<212>  DNA
<213>  Cucumis melo

<400>  1
ggttcttaat taatattcaa tttgtcataa actagccatt taagattcac agtgtcaaga      60

ttatcaactt tgccagtcaa acaaccacaa catggtttaa actatccatt caactttaaa     120

ataaacagtt aacttactta tgaggctatt ttacaaaaat aacatttcaa tttataaaaa     180

aataacattt catttttaaa caacccctat tgatgaaata tatatatatc gaacctttca     240

tttttattgg tggttaatta tcacttaaaa aaagtagtgt gttggtaaaa aagtaaatga     300

aatttatata caagaatgga ttaaagaaaa accatctcca cccaattatt ttatgtaaaa     360

aagaatccat ccctaatgta aacataggtg ttatgagagt gaatcacgtg tcttgatgtg     420

tctcacttct ttaatattta atatatatgt gtgtgtgtgt gtgtgtgttt tttttttttg     480

gaagtaaaat aaaatttatt actcaatact ccaaaaaggg attccatttc caactcaaaa     540

attaattaat tagttaatta attaattaat tatgaagaga aacccacacc catgacaaag     600

ataaaagcgg gcacaaatgt gattacacaa aaaataggag gcacttctgg taaaatggta     660

aaataaattc tccccatatt ccaaaattcc tcaatatatt ttcattttat tcccctctat     720

aaataagttg caaagcaatt cccgtgtaag ccatcccccc aaacttctct caaacatatc     780

cacataccaa aaacaaaaac aaaaaaaact aaaaagatcc accgccggtg acaatggccg     840

ccgcgaatct tcgaccggac gccgaaaact cattactctc ttcccatctc tacaaccccg     900

aagtttacac ccaagtgtta ccccaacaag gtagtaatta attaagtttc ttacaaaatt     960

tcatctttta aatttttttt ttttttgttt atttgttttt aattttcaaa tttatgtttt    1020

taggtacggg agagggaaat aagccgacga agcggcggag gaggaggagc aaagcgaagg    1080

agggaggcgg gggggcgggg tcgttgaaga agaggaagct gagttcagag caagtgaagc    1140

ttttggagat gaattttggg aatgaacata aattggaatc ggagaggaaa gatcggttgg    1200

cttctgaatt gggacttgat ccacggcagg tggcggtttg gtttcagaac cgccgtgcac    1260

ggtggaagaa taagaagctg gaagaggaat attccactct taaaaaagct catgattctg 1320

ttcttctaca aaaatcccat cttgaatctg aggtcagttt tattacaatt tatttttctt 1380

tcttttttgg tatatttttc tctccaaaaa aattacgtac ggtcagagag ctataattgt 1440

ttgaccttat cgttaatttc atagtactct aaataaactg gattttgatt tgcttatcta 1500

gtaagaaatg aacgaaattt ggtaactaaa taacaatgtt ttctttttcc cctaaggaat 1560

aaaaaagaag aacgtgcatg agctcttggc tcgaagaata tgatgccacc ttatagggtt 1620

tggaatttgt gaaataataa atagtataaa aaatcaattt cttccctgaa aaaactcaga 1680

aactttattt ttcttttata ttttttttcc cattaacgta cagcttactg ccatctgagg 1740

caaacacaaa tgggcataac aacctctctc ttctctcctt aaagaacgat ccgtacacca 1800

cataatatat atatgtatat atataactcg ataacatttc ttataataat ttgtttggtt 1860

ggattttagt tggtgaaagt gaaagagcaa ttgaaagaag caaagaacga gatacgaaaa 1920

atggtagaag gaagtgaggt gagaaataat tcaagcaata gcccaagctc ttcagtgaca 1980

atggaagcag ttgaggaagc agcagtagtt cctttgggag agcttttctt tgaagaatat 2040

gaagatgtat tctattgcat gcaagacaac aattacaatc aaggattgga ctgggcgctt 2100

aatcttaatt ttatgtagat tagttatttt tattatatct aactattaat cactctcttt 2160

taatttagtt tgtagatata gaactctcat agattatatc agatctctag ctaattatat 2220

gtacatttct ctccctctct ctctctctct ctctctctct ctctctaact agtgttattt 2280

tccagaactt ggaattaatt aattgtgtta ttatgtctct ttttggtctc actatgttgg 2340

gctctccttt tttaaccttt ttcttttctc ataccatgtg cttttttttt tcttaaaacc 2400

ccaaaatata ccaacattta aaaattatct caaacttatg cttataaaac cctattttga 2460

ctaattaatc attttctttt aacgacatat aatctggcta atttctttta atttattttt 2520

ctctcccgca agttgaaaat agattaaatt agagacgtga ttccaatttc tattaacgaa 2580

agatcgtgtc tatgctaact aagtaattat actccaa 2617

<210> 2
<211> 675
<212> DNA
<213> Cucumis melo

<400> 2
atggccgccg cgaatcttcg accggacgcc gaaaactcat tactctcttc ccatctctac 60

aaccccgaag tttacaccca agtgttaccc caacaaggta cgggagaggg aaataagccg 120

acgaagcggc ggaggaggag gagcaaagcg aaggagggag gcgggggggc ggggtcgttg 180

aagaagagga agctgagttc agagcaagtg aagcttttgg agatgaattt tgggaatgaa 240

cataaattgg aatcggagag gaaagatcgg ttggcttctg aattgggact tgatccacgg 300

caggtggcgg tttggtttca gaaccgccgt gcacggtgga agaataagaa gctggaagag 360

gaatattcca ctcttaaaaa agctcatgat tctgttcttc tacaaaaatc ccatcttgaa 420

tctgagttgg tgaaagtgaa agagcaattg aaagaagcaa agaacgagat acgaaaaatg 480

gtagaaggaa gtgaggtgag aaataattca agcaatagcc caagctcttc agtgacaatg 540

gaagcagttg aggaagcagc agtagttcct ttgggagagc ttttctttga agaatatgaa 600

gatgtattct attgcatgca agacaacaat tacaatcaag gattggactg ggcgcttaat 660

cttaatttta tgtag 675

<210> 3
<211> 224
<212> PRT
<213> Cucumis melo

<400> 3

Met Ala Ala Ala Asn Leu Arg Pro Asp Ala Glu Asn Ser Leu Leu Ser
1               5                   10                  15

Ser His Leu Tyr Asn Pro Glu Val Tyr Thr Gln Val Leu Pro Gln Gln
            20                  25                  30

Gly Thr Gly Glu Gly Asn Lys Pro Thr Lys Arg Arg Arg Arg Arg Ser
        35                  40                  45

Lys Ala Lys Glu Gly Gly Gly Gly Ala Gly Ser Leu Lys Lys Arg Lys
    50                  55                  60

Leu Ser Ser Glu Gln Val Lys Leu Leu Glu Met Asn Phe Gly Asn Glu
65                  70                  75                  80

His Lys Leu Glu Ser Glu Arg Lys Asp Arg Leu Ala Ser Glu Leu Gly
                85                  90                  95

Leu Asp Pro Arg Gln Val Ala Val Trp Phe Gln Asn Arg Arg Ala Arg
            100                 105                 110

Trp Lys Asn Lys Lys Leu Glu Glu Glu Tyr Ser Thr Leu Lys Lys Ala
        115                 120                 125

His Asp Ser Val Leu Leu Gln Lys Ser His Leu Glu Ser Glu Leu Val
    130                 135                 140

Lys Val Lys Glu Gln Leu Lys Glu Ala Lys Asn Glu Ile Arg Lys Met
145                 150                 155                 160

Val Glu Gly Ser Glu Val Arg Asn Asn Ser Ser Asn Ser Pro Ser Ser

165                 170                 175

Ser Val Thr Met Glu Ala Val Glu Glu Ala Ala Val Val Pro Leu Gly
            180                 185                 190

Glu Leu Phe Phe Glu Glu Tyr Glu Asp Val Phe Tyr Cys Met Gln Asp
        195                 200                 205

Asn Asn Tyr Asn Gln Gly Leu Asp Trp Ala Leu Asn Leu Asn Phe Met
    210                 215                 220

<210>  4
<211>  112
<212>  PRT
<213>  Cucumis melo

<400>  4

Met Ala Ala Ala Asn Leu Arg Pro Asp Ala Glu Asn Ser Leu Leu Ser
1               5                   10                  15

Ser His Leu Tyr Asn Pro Glu Val Tyr Thr Gln Val Leu Pro Gln Gln
            20                  25                  30

Gly Thr Gly Glu Gly Asn Lys Pro Thr Lys Arg Arg Arg Arg Arg Ser
        35                  40                  45

Lys Ala Lys Glu Gly Gly Gly Gly Ala Gly Ser Leu Lys Lys Arg Lys
    50                  55                  60

Leu Ser Ser Glu Gln Val Lys Leu Leu Glu Met Asn Phe Gly Asn Glu
65                  70                  75                  80

His Lys Leu Glu Ser Glu Arg Lys Asp Arg Leu Ala Ser Glu Leu Gly
                85                  90                  95

Leu Asp Pro Arg Gln Val Ala Val Trp Phe Gln Asn Arg Arg Ala Arg
            100                 105                 110

<210>  5
<211>  224
<212>  PRT
<213>  Cucumis sativus

<400>  5
   Met Ala Ala Val Asn Leu Arg Pro Asp Ala Asp Asn Ser Leu Leu Val
   1               5                   10                  15
   Ser His Leu Tyr Asn Pro Glu Val Tyr Thr Gln Gln Val Leu Pro Gln
               20                  25                  30
   Gln Gly Thr Gly Glu Gly Ser Lys Pro Thr Lys Arg Arg Arg Arg Arg
           35                  40                  45
   Ser Lys Ala Lys Glu Gly Gly Gly Ala Ala Gly Leu Lys Lys Arg Lys
       50                  55                  60
   Leu Ser Ser Glu Gln Val Lys Leu Leu Glu Met Asn Phe Gly Asn Glu
   65                  70                  75                  80
   His Lys Leu Glu Ser Glu Arg Lys Asp Arg Leu Ala Ser Glu Leu Gly
                   85                  90                  95
   Leu Asp Pro Arg Gln Val Ala Val Trp Phe Gln Asn Arg Arg Ala Arg
               100                 105                 110
   Trp Lys Asn Lys Lys Leu Glu Glu Glu Tyr Ser Thr Leu Lys Lys Ala
           115                 120                 125
   His Asp Ser Val Val Leu Gln Lys Ser His Leu Glu Ser Glu Leu Met
       130                 135                 140
   Lys Val Lys Glu Gln Leu Lys Glu Ala Lys Asn Glu Ile Arg Lys Met
   145                 150                 155                 160
   Val Glu Gly Ser Glu Val Arg Asn Asn Ser Ser Asn Ser Pro Ser Ser
                   165                 170                 175
   Ser Val Thr Met Glu Ala Val Glu Glu Ala Ala Val Val Pro Leu Gly
               180                 185                 190
   Glu Leu Phe Phe Glu Glu Tyr Glu Asp Val Phe Tyr Cys Met Gln Asp
           195                 200                 205
   Asn Asn Tyr Asn Gln Gly Leu Asn Trp Ala Leu Asn Leu Asn Phe Met
       210                 215                 220

<210>  6
<211>  106
<212>  PRT
<213>  Cucumis sativus

<400>  6

Met Ala Ala Val Asn Leu Arg Pro Asp Ala Asp Asn Ser Leu Leu Val
1               5                   10                  15

Ser His Leu Tyr Asn Pro Glu Val Tyr Thr Gln Gln Val Leu Pro Gln
            20                  25                  30

Gln Gly Thr Gly Glu Gly Ser Lys Pro Thr Lys Arg Arg Arg Arg Arg
        35                  40                  45

Ser Lys Ala Lys Glu Gly Gly Gly Ala Ala Gly Leu Lys Lys Arg Lys
    50                  55                  60

Leu Ser Ser Glu Gln Val Lys Leu Leu Glu Met Asn Phe Gly Asn Glu
65                  70                  75                  80

His Lys Leu Glu Ser Glu Arg Lys Asp Arg Leu Ala Ser Glu Leu Gly
                85                  90                  95

Leu Asp Pro Arg Gln Val Ala Val Trp Phe
            100                 105

<210>  7
<211>  112
<212>  PRT
<213>  Cucumis sativus

<400>  7

Met Ala Ala Val Asn Leu Arg Pro Asp Ala Asp Asn Ser Leu Leu Val
1               5                   10                  15

Ser His Leu Tyr Asn Pro Glu Val Tyr Thr Gln Gln Val Leu Pro Gln
            20                  25                  30

Gln Gly Thr Gly Glu Gly Ser Lys Pro Thr Lys Arg Arg Arg Arg Arg
        35                  40                  45

Ser Lys Ala Lys Glu Gly Gly Gly Ala Ala Gly Leu Lys Lys Arg Lys
    50                  55                  60

Leu Ser Ser Glu Gln Val Lys Leu Leu Glu Met Asn Phe Gly Asn Glu
65                  70                  75                  80

His Lys Leu Glu Ser Glu Arg Lys Asp Arg Leu Ala Ser Glu Leu Gly
                85                  90                  95

Leu Asp Pro Arg Gln Val Ala Val Trp Phe Gln Asn Arg Arg Ala Arg
            100                 105                 110

<210>  8
<211>  220
<212>  PRT
<213>  Citrullus lanatus

<400>  8

Met Ala Ala Met Asn Leu Arg Pro Asp Gly Asp Asn Ser Gln Leu Ile
1               5                   10                  15

Ser His Leu Tyr Asn Pro Glu Ile Tyr Thr Gln Met Leu Pro Gln Gln
            20                  25                  30

Gly Thr Ala Glu Gly Asn Lys Pro Pro Lys Arg Arg Arg Arg Arg Ser
        35                  40                  45

Lys Ala Lys Glu Gly Gly Gly Ala Gly Ala Ala Gly Leu Lys Lys Arg
    50                  55                  60

Lys Leu Ser Ser Glu Gln Val Lys Leu Leu Glu Ile Asn Phe Gly Asn
65                  70                  75                  80

Glu His Lys Leu Glu Ser Glu Arg Lys Asp Arg Leu Ala Ser Glu Leu
                85                  90                  95

Gly Leu Asp Pro Arg Gln Val Ala Val Trp Phe Gln Asn Arg Arg Ala
            100                 105                 110

Arg Trp Lys Asn Lys Lys Leu Glu Glu Glu Tyr Ser Thr Leu Lys Lys
        115                 120                 125

Ala His Asp Ser Val Leu Leu Gln Asn Ser His Leu Glu Ser Glu Leu
    130                 135                 140

Val Lys Val Lys Glu Gln Leu Lys Glu Ala Lys Asn Glu Ile Lys Lys
145                 150                 155                 160

Leu Gly Glu Gly Ser Glu Arg Ile Ser Ser Asn Asn Ser Pro Ser Ser
                165                 170                 175

Ser Val Thr Met Glu Ala Val Glu Ala Val Pro Leu Gly Glu Leu Phe
            180                 185                 190

Leu Glu Glu Tyr Glu Asp Val Phe Tyr Cys Met Gln Asp Asn Asn Tyr
        195                 200                 205

Asn Gln Gly Met Asp Trp Leu Asn Leu Asn Tyr Met
    210                 215                 220

<210>  9
<211>  217
<212>  PRT
<213>  Cucurbita pepo

<400>  9

Met Ala Ala Val Asn Leu Arg Pro Asp Asp Asp Asn Ser Thr Leu Ile
1               5                   10                  15

Ser His Ile Tyr Asn Pro Glu Ile Tyr Thr Gln Met Leu Pro Gln Gln
            20                  25                  30

Gly Met Ala Glu Gly Asn Lys Pro Thr Lys Arg Arg Arg Arg Arg Ser
        35                  40                  45

Arg Ala Lys Glu Val Ala Glu Ala Ala Ala Gly Leu Lys Lys Arg Lys
    50                  55                  60

Leu Ser Ser Glu Gln Val Lys Leu Leu Glu Ile Asn Phe Gly Asn Glu
65                  70                  75                  80

His Lys Leu Glu Thr Glu Arg Lys Asn Arg Leu Ala Ser Glu Leu Gly
                85                  90                  95

Leu Asp Pro Ser Gln Val Ala Ile Trp Phe Gln Asn Arg Arg Ala Arg
            100                 105                 110

Trp Lys Asn Gln Lys Leu Glu His Gln Tyr Ser Ile Leu Lys Thr Ala
        115                 120                 125

His Asp Ser Ala Val Leu Gln Lys Thr His Leu Gln Ser Glu Ile Glu
    130                 135                 140

Lys Leu Lys Glu Gln Leu Ala Glu Ala Lys Asn Glu Ile Gln Lys Leu
145                 150                 155                 160

Val Glu Val Ser Glu Arg Asn Arg Arg Trp Ser Val Thr Met Asp Asp
                165                 170                 175

Val Gln Ala Ala Ala Cys Trp Gly Glu Ile Leu Met Glu Glu Tyr Glu
            180                 185                 190

Asp Val Phe Tyr Ser Met Gln Glu Asn Asn Tyr Asn Gln Pro Val Glu
        195                 200                 205

Trp Tyr Leu Asn Tyr Met Tyr Thr Asn
    210                 215

<210>  10
<211>  216
<212>  PRT
<213>  Cucurbita pepo

<400>  10

Met Ala Ala Ala Asp Asp Asp Asn Thr Leu Leu Ile Ser Asn Leu Tyr
1               5                   10                  15

Ser Pro Gly Val Tyr Thr Gln Ile Leu Pro Gln Gln Gly Ala Ala Glu
            20                  25                  30

Gly Asn Lys Pro Thr Lys Arg Arg Arg Arg Arg Ser Lys Ala Lys Glu
        35                  40                  45

Ala Ala Gly Ala Ala Gly Leu Lys Lys Arg Lys Leu Ser Thr Glu Gln
    50                  55                  60

Val Lys Leu Leu Glu Met Asn Phe Gly Asp Glu His Lys Leu Glu Ser
65                  70                  75                  80

Glu Arg Lys Asp Arg Leu Ala Ser Glu Leu Gly Leu Asp Pro Arg Gln
                85                  90                  95

Val Ala Val Trp Phe Gln Asn Arg Arg Ala Arg Trp Lys Asn Lys Lys
            100                 105                 110

Leu Glu Glu Glu Tyr Ser Thr Leu Lys Lys Ala His Asp Ser Ile Ile
        115                 120                 125

Leu Gln Asn Ser His Leu Glu Tyr Glu Met Thr Lys Leu Ser Glu Gln
    130                 135                 140

Leu Arg Glu Ala Lys Asn Glu Ile Gln Arg Leu Val Glu Gly Ser Glu
145                 150                 155                 160

Lys Ile Trp Ser Asn Ser Pro Thr Ser Ser Glu Thr Met Asp Ala Val
                165                 170                 175

Pro Pro Pro Ala Pro Ser Trp Gly Glu Leu Phe Leu Gly Glu Tyr Glu
            180                 185                 190

Asp Val Phe Tyr Cys Met Gln Gln Gln His Gly Asn Tyr Ser Gln Gly
        195                 200                 205

Met Glu Trp Leu Asn Leu Tyr Met
    210                 215

<210>  11
<211>  220
<212>  PRT
<213>  Lagenaria siceraria

<400>  11

Met Ala Ala Val Asn Leu Arg Pro Asp Gly Asp Asn Ser Leu Leu Ile
1               5                   10                  15

Ser His Leu Tyr Asn Pro Glu Ile Tyr Thr Gln Met Leu Pro Gln Gln
            20                  25                  30

Gly Thr Ala Glu Gly Asn Lys Pro Pro Lys Arg Arg Arg Arg Arg Ser
        35                  40                  45

Lys Ala Lys Glu Gly Gly Gly Ala Gly Ala Ala Gly Leu Lys Lys Arg
    50                  55                  60

Lys Leu Ser Ser Glu Gln Val Lys Leu Leu Glu Leu Asn Phe Gly Asn
65                  70                  75                  80

Glu His Lys Leu Glu Ser Glu Arg Lys Asp Arg Leu Ala Ser Glu Leu
                85                  90                  95

Gly Leu Asp Pro Arg Gln Val Ala Val Trp Phe Gln Asn Arg Arg Ala
            100                 105                 110

Arg Trp Lys Asn Lys Lys Leu Glu Glu Glu Tyr Ser Thr Leu Lys Lys
        115                 120                 125

Ala His Asp Ser Val Leu Leu Gln Lys Ser His Leu Glu Ser Glu Met
    130                 135                 140

Val Arg Leu Lys Glu Gln Leu Thr Glu Ala Lys Asn Glu Ile Lys Lys
145                 150                 155                 160

Leu Val Glu Gly Ser Glu Arg Ile Ser Ser Asn Asn Ser Pro Ser Ser
                165                 170                 175

Ser Val Thr Met Glu Ala Val Glu Ala Val Pro Leu Gly Glu Leu Phe
            180                 185                 190

Leu Glu Glu Tyr Glu Asp Val Phe Tyr Cys Met Gln Asp Asn Asn Tyr
        195                 200                 205

Asn Gln Gly Met Asp Trp Leu Asn Leu Asn Tyr Met
    210                 215                 220

<210>  12
<211>  25
<212>  DNA
<213>  Cucumis melo

<400>  12

gaaggacccg taagaatata atgtc                                              25

<210>  13
<211>  25
<212>  DNA
<213>  Cucumis melo

<400>  13
ggggcatttt gcctaatttt ggtga                                              25

<210>  14
<211>  21
<212>  DNA
<213>  Cucumis melo

<400>  14
tattgagaat gactgtcact t                                                  21

<210>  15
<211>  20
<212>  DNA
<213>  Cucumis melo

<400>  15
cttcactatc ttgcctttcc                                                    20

<210>  16
<211>  27
<212>  DNA
<213>  Cucumis melo

<400>  16
aggcaaacac aaatgggcat aacaacc                                            27

<210>  17
<211>  21
<212>  DNA
<213>  Cucumis melo

<400>  17
gtaattgttg tcttgcatgc a                                                  21

<210>  18
<211>  25
<212>  DNA
<213>  Cucumis melo

<400>  18
agtgtgtgag atgatggatg atgtg                                              25

<210>  19
<211>  22
<212>  DNA
<213>  Cucumis melo

<400>  19

tgccaaactt gagaaagagt tg                                                22

<210>  20
<211>  25
<212>  DNA
<213>  Cucumis melo

<400>  20
acaaaccgaa ctttatgagg taggc                                             25

<210>  21
<211>  25
<212>  DNA
<213>  Cucumis melo

<400>  21
tgactcgatg aaatgggaag cttgt                                             25

<210>  22
<211>  25
<212>  DNA
<213>  Cucumis melo

<400>  22
gagagtgaat cacgtgtctt gatgt                                             25

<210>  23
<211>  25
<212>  DNA
<213>  Cucumis melo

<400>  23
agttggaaat ggaatccctt tttgg                                             25

<210>  24
<211>  20
<212>  DNA
<213>  Cucumis melo

<400>  24
aattcccgtg taagccatcc                                                   20

<210>  25
<211>  20
<212>  DNA
<213>  Cucumis melo

<400>  25
caaagttcat ctccaaaagc                                                   20

<210>  26
<211>  20
<212>  DNA
<213>  Cucumis melo

<400>  26

tacgggagag ggaaataagc                                                    20

<210>  27
<211>  20
<212>  DNA
<213>  Cucumis melo

<400>  27
acaattatag ctctctgacc                                                    20

<210>  28
<211>  20
<212>  DNA
<213>  Cucumis melo

<400>  28
cacaaatggg cataacaacc                                                    20

<210>  29
<211>  19
<212>  DNA
<213>  Cucumis melo

<400>  29
tatgagagtt ctatatcta                                                     19

<210>  30
<211>  20
<212>  DNA
<213>  Cucumis sativus

<400>  30
ctcaaaccta tccacatacc                                                    20

<210>  31
<211>  20
<212>  DNA
<213>  Cucumis sativus

<400>  31
cctctccgat tccaatttat                                                    20

<210>  32
<211>  20
<212>  DNA
<213>  Cucumis sativus

<400>  32
ggagatgaat tttgggaatg                                                    20

<210>  33
<211>  20
<212>  DNA
<213>  Cucumis sativus

<400>  33

taactctctg accgtacgta                                                    20

<210>  34
<211>  20
<212>  DNA
<213>  Cucumis sativus

<400>  34
ctgccatctg aggcaaatat                                                    20

<210>  35
<211>  20
<212>  DNA
<213>  Cucumis sativus

<400>  35
ttaagctaga gatgatctaa                                                    20